# EUROPEAN PATENT SPECIFICATION

(11) **EP 2 550 535 B1**
(45) Date of publication and mention of the grant of the patent: **06.05.2015**
(21) Application number: 11710137.8
(22) Date of filing: 24.03.2011
(51) Int. Cl.: G01N 33/68, G01N 33/72

(54) **HBF AND A1M AS EARLY STAGE MARKERS FOR PREECLAMPSIA**
HBF UND A1M ALS MARKERS ZUR FRÜHERKENNUNG VON PREECLAMPSIA
HBF ET A1M UTILISEES COMME MARQUEURS PATHOLOGIQUES DE PREECLAMPSIA A UN STADE PRECOCE

(30) Priority: 24.03.2010 DK 201000245
(43) Date of publication of application: 30.01.2013
(73) Proprietor: Preelumina Diagnostics AB, 223 81 Lund (SE)
(72) Inventor: AKERSTRÖM, Bo, 227 38 Lund (SE); OLSSON, Magnus, 273 71 Lövestad (SE); HANSSON, Stefan, 234 42 Lomma (SE)
(74) Representative: Chas. Hude A/S
(86) International application number: PCT/EP2011/001458
(87) International publication number: WO 2011/116958

(56) References cited:
- WO-A1-2008/098734
- WO-A1-2009/108073
- OLSSON M G ET AL: "Increased levels of cell-free hemoglobin, oxidation markers, and the antioxidative heme scavenger +/-1-microglobulin in preeclampsia", FREE RADICAL BIOLOGY AND MEDICINE, ELSEVIER SCIENCE, US, vol. 48, no. 2, 15 January 2010 (2010-01-15), pages 284-291, XP026820721, ISSN: 0891-5849 [retrieved on 2009-10-30]
- HOLZGREVE W ET AL: "Fetal cells in cervical mucus and maternal blood", BAILLIERE'S BEST PRACTICE AND RESEARCH. CLINICAL OBSTETRICS ANDGYNAECOLOGY, BAILLIERE TINDALL, LONDON, GB LNKD- DOI:10.1053/BEOG.1999.0106, vol. 14, no. 4, 1 January 2000 (2000-01-01), pages 709-722, XP008081646, ISSN: 1521-6934
- PATERNOSTER D M ET AL: "Predictive markers of pre-eclampsia in hypertensive disorders of pregnancy", INTERNATIONAL JOURNAL OF GYNECOLOGY AND OBSTETRICS, vol. 66, no. 3, September 1999 (1999-09), pages 237-243, XP002604482, ISSN: 0020-7292

## Description

### TECHNICAL FIELD

Present invention relates to the use of fetal hemoglobin (HbF) and (A1M) as markers for preeclampsia (PE).

### BACKGROUND OF THE INVENTION

Preeclampsia (PE) affects up to 7-15 % of all pregnancies and is an important factor of maternal morbidity and mortality. The clinical manifestations of PE appear in the second to third trimester. Early onset PE, the more severe form, appears before 34 gestational weeks (GW). The symptoms are often vague and diverse such as headache, blurry vision and edema. Hypertension and proteinurea are not only hallmarks of the disease, but are also integral to the diagnosis.

PE is generally believed to develop in two stages, where the first stage is characterized by defective placentation, which leads to uneven blood perfusion, ischemic re-perfusion injuries and increased oxidative stress in the placenta. The second stage of PE, the maternal syndrome, is characterized by a general vascular dysfunction based on severe endothelial damage that eventually causes vasoconstriction, general vascular inflammation and multiple organ dysfunction.

Although there are clinical parameters used for diagnosing PE, there are great difficulties in clinical diagnosis of PE mainly due to the vague symptoms. Neither the amount of proteinurea nor the level of hypertension correlate very well with the severity of PE as examined by Schroeder BM. ACOG practice bulletin on diagnosing and managing preeclampsia and eclampsia, 15;66(2):330-1 and Redman CW & Sargent IL, Science ;308(5728):1592-4).

Due to the fact that there are no reliable biomarkers for predicting and/or diagnosing PE, great effort has lately been put into this field. However, finding a good biomarker is a major challenge. Many of the suggested markers need to be combined with each other and/or evaluated in combination with Doppler ultrasound to improve the diagnostic accuracy. Up to date, several markers have been suggested, but none are accepted as being useful biomarkers for the clinical prediction or diagnosis of PE. Two anti-angiogenic factors are promising, showing a significant association with PE: soluble fms-like tyrosine kinase 1 (sFlt) and soluble endoglin as discussed and reviewed in Baumann et al., Molecular aspects of medicine 2007 Apr;28(2):227-44, Grill et al., Reprod Biol Endocrinol 2009;7:70, Levine et al,. The New England journal of medicine 2004 Feb 12;350(7):672-83 and in Papageorghiou et al., Current opinion in obstetrics & gynecology 2006 Dec;18(6):594-600.

Some methods of diagnosis have been described in the prior art but none of these have yet been successfully used in the clinic on a large scale. For example can be mentioned: US 5,079,171 and US 5,108,898, which disclose that PE, pregnancy-induced hypertension, and eclampsia can be diagnosed by identifying the presence of an endothelial cell marker, cellular fibronectin, in a sample of blood, plasma or serum of a pregnant woman, for example, by using a sandwich or competition immunoassay. The cellular fibronectin derives from endothelial cells that are ruptured or disturbed during the disease process.

Several studies have shown a prediction rate around 50% particularly for early onset PE, using Doppler ultrasound.

The anti-angiogenic marker sFlt is a well-described potential PE marker when used in the second and third trimester. However, in the first trimester, sFlt levels are unchanged compared to normal pregnancies, making it less useful for prediction of PE. The placenta growth factor (PIGF) has also been proposed as a PE marker, but available data show conflicting results. Endoglin has a lower screening efficiency in early pregnancy when compared to the predictive values obtained when the combination sFlt/PIGF were used.

US 5,238,819 discloses the diagnosis of PE using an assay to measure a mitogenic factor in blood. The mitogenic factor is a proteinaceous compound of about 160 kDa and is capable of stimulating fibroblast mitosis. Its presence is detected by detecting radiolabelled thymidine uptake by cells activated by the sera or plasma of a PE subject. This marker appears after damage to the maternal vascular bed already has occurred, hence late in the disease progression.

WO 05/093413 (Yale University and Brigham and Women's Hospital) discloses a method of diagnosing of severe PE in a pregnant woman, comprising measuring the level of free hemoglobin (Hb) in a cerebrospinal fluid sample.

Olsson et al. (Free Radical Biology and Medicine, vol. 48, 2010, 284-291) describes measurements of HbA, HbF and A1M in plasma and urine. Samples were collected from pregnant women just before delivery.

WO 2008/098734 discloses the use of HbF for diagnosing preeclampsia.

Currently, there are no known cures for PE. PE can vary in severity from mild to life threatening. A mild form of PE can remain mild with bed rest and frequent monitoring. For moderate to severe cases, hospitalization is necessary and blood pressure medication and anticonvulsant medications to prevent seizures are prescribed. If the condition becomes life-threatening to the mother or the baby, the only cure is to terminate the pregnancy often resulting in a pre-term delivered baby.

Clearly, the lack of therapy for PE coupled with the age-old lack of ability to diagnose it until the disease has progressed to a more severe form, prompts the need for novel approaches for diagnosing and treating PE, which is a significant public health problem.

### SUMMARY OF THE INVENTION

The invention as disclosed herein is based on data from gene and protein profiling studies which have revealed increased synthesis and accumulation of fetal hemoglobin (HbF) in the vascular lumen of placenta obtained from women with PE. Further, the inventors have found that the maternal plasma and/or serum concentrations of HbF and the heme scavenger α1-microglobulin (A1 M) correlate with severity of the disease in term pregnancies.

As disclosed herein, the inventors have surprisingly found that the combination of the HbF-ratio and A1M levels provides an extraordinary precise prediction and may be applied as bio-markers for early screening of PE.

The invention provides a method for predicting the risk of developing preeclampsia, the method being characterized by comprising the steps
a) during gestational week 10-20 measuring the levels of A1 M, HbA and HbF in an isolated serum sample from a subject,
b) calculating the ratio HbF/total Hb (HbF ratio)
wherein the subject is considered to have an elevated probability of developing preeclampsia if the measured HbF-ratio exceeds 0.0020 and the measured A1M level exceeds 21 µg/ml.

The invention also provides use of A1 M, HbA and HbF as early stage markers during gestational week 10-20 for predicting the possibility of a subject to develop preeclampsia, wherein the subject is considered to have an elevated probability of developing preeclampsia if the ratio HbF/total Hb (HbF-ratio) exceeds 0.0020 and the measured A1 M level exceeds 21 µg/ml.

### DETAILED DESCRIPTION OF THE INVENTION

Studies conducted by the inventors have indicated the involvement of Hb-induced oxidative stress in the development of PE. Up-regulation of the HbF genes and the accumulation of the protein in the PE placenta have been observed. Free Hb, i.e. outside the red blood cell, and its metabolites heme and iron induce oxidative stress by formation of reactive oxygen species (ROS). The oxidative stress may damage the blood-placenta barrier, leading to leakage of HbF into the maternal circulation and eventually cause elevated levels in the maternal plasma or serum. Indeed, increased levels of HbF, total Hb and markers for oxidative stress, as well as the heme-scavenger and anti-oxidant endogenous protein, A1M, were found to be elevated in both plasma and placenta from women with PE.

A1 M is an important endogenous scavenger for heme. Its expression has been shown to be up-regulated in response to free Hb and ROS. A1 M is synthesized and secreted mostly from the liver, and rapidly distributed to different tissues where it is found in the extra-vascular compartments both in free form and as high molecular weight complexes bound to IgA, albumin and prothrombin. The biological and metabolical link between Hb and A1M is further supported by our current results, since both HbF and A1 M were elevated in the first trimester samples. The simultaneous increase of A1M and Hb has also been shown in PE in term pregnancies.

As disclosed herein, the concentration of A1 M and one or more of the markers HbA, HbF or the HbF ratio (Total Hb/HbF) may be used to predict the risk of developing PE. The predictive values obtained for the HbF-ratio and A1 M levels, especially in combination, compare favorably with most other promising PE bio-markers. Furthermore, HbF-ratio and A1M levels are also useful for PE diagnosis and determination of PE severity in term pregnancies.

An aspect of the invention relates to the use of the HbF and A1M-level in combinations for diagnosing PE as claimed in claim 1 or 13. Hence this aspect of the invention relates to a method for predicting the risk of developing PE by measuring the concentration of HbF and A1 M (the HbF and A1 M level) in a subject and relating the measured levels to reference-values in order to assess the risk of developing PE. The concentration may be measured on samples including urine-, blood-, serum- or plasma-, saliva- and most preferably on human plasma-samples. The HbF and A1M level may be measured by any applicable method including chromatography, mass spectrometry and most preferably by enzyme linked immunosorbent arrays i.e. ELISA for HbF and radioimmunoassay (RIA) for A1M.

The invention relates to the use of the HbF ratio with the A1M-level for diagnosing PE as claimed in claim 1. To correct for the contribution of HbF derived from lysed maternal red blood cells, the ratio HbF/total Hb (HbF-ratio) was used. Even though care should be taken to minimize technical hemolysis during sample processing, it often happens. By using the HbF-ratio, most samples can be evaluated without risk of a false positive prediction or diagnosis. The HbF-ratio is correlated to the A1M level and thereby provides a more precise measure, applicable for predicting the risk of developing PE. Hence this aspect of the invention relates to a method for predicting the risk of developing PE by measuring the concentration of HbA, HbF and A1 M (the HbA, HbF and A1M level) in a subject, calculating the HbF ratio and correlating the calculated HbF ratio with the A1M level to a scheme or reference-values in order to provide an indication of the risk of developing PE. The mathematical analysis may be performed by any suitable method, including regression analysis or logistic regression analyses as e.g. binary logistic regression analyses. The concentration may be measured on samples including urine, blood, serum, saliva and most preferably on human plasma. The HbF and A1M level may be measured by any applicable method including chromatography, mass spectrometry and most preferably by enzyme linked immunosorbent arrays i.e. ELISA for HbF and radioimmunoassay (RIA) or ELISA for A1M.

Described herein is a method for diagnosing or predicting the risk of developing PE, the method being characterized by comprising the steps:
a) Measuring the level of A1 M and one or more of the compounds HbA, HbF in a subject or in an isolated sample from a subject
b) Comparing the measured levels with one or more reference values to assess the probability of developing PE.

As mentioned above, the combined information relating to A1 M and HbF levels give advantages compared with information relating to HbF alone.

The invention provides a method for predicting the risk of developing preeclampsia, the method being characterized by comprising the steps
a) during gestational week 10-20 measuring the levels of A1 M, HbA and HbF in an isolated serum sample from a subject,
b) calculating the ratio HbF/total Hb (HbF ratio)
wherein the subject is considered to have an elevated probability of developing preeclampsia if the measured HbF-ratio exceeds 0.0020 and the measured A1M level exceeds 21 µg/ml.

The invention also provides use of A1M, HbA and HbF as early stage markers during gestational week 10-20 for predicting the possibility of a subject to develop preeclampsia, wherein the subject is considered to have an elevated probability of developing preeclampsia if the ratio HbF/total Hb (HbF-ratio) exceeds 0.0020 and the measured A1 M level exceeds 21 µg/ml.

The method is characterized by comprising the steps
a) Measuring the level of A1 M, HbA and HbF in a subject or in an isolated sample from a subject
b) Calculating the HbF ratio.
c) Comparing the HbF-ratio and the A1 M concentration to reference values to assess the probability of developing preeclampsia in accordance with claim 1.

As a further development, mathematical methods and analyses may be employed, in which the measured values are statistically analysed, thereby providing a method for diagnosing or predicting the risk of developing PE, the method being characterized by comprising the steps:
a) Measuring the level of A1 M and one or more of the compounds HbA, HbF in a subject or in an isolated sample from a subject
b) Calculating the HbF ratio
c) Correlating the HbF ratio to the A1M level
d) Comparing the ratio as calculated in b), the correlation data as calculated in c) or levels measured in a) to one or more reference values to assess the probability of developing PE.

Present method(s) may further include statistical analyses of the measured values as explained herein. Hence the diagnosis of, or risk of developing PE may be assessed by analyzing the A1M level and the HbF-ratio and/or the HbF, HbA or A1M-levels as separate measures, using a binary logistic regression analysis, optionally with a likelihood ratio (LR) test.

To assess the risk of developing PE, the measured and/or calculated values may be interpreted and compared to sets of reference values.

By measuring and analyzing the HbF level in a subject, the risk of developing PE may be assed. A subject is considered to have an elevated probability of developing PE if the measured HbF level exceeds 0.45 µg/ml, such as e.g. 0.75 µg/ml such as e.g. 1.0 µg/ml, such as e.g. 1.5 µg/ml such as e.g. 2.5 µg/ml.

By measuring and analyzing the A1M level in a subject, the risk of developing PE may be assed. A subject is considered to have an elevated probability of developing PE if the measured A1M level A1M-level exceeds 21µg/ml, such as e.g. 23µg/ml, such as e.g. 25µg/ml, such as e.g. 30µg/ml, such as e.g. 35µg/ml, such as e.g. 40µg/ml.

By measuring and analyzing the HbA and the HbF level in a subject, the risk of developing PE may be assed by calculating the HbF-ratio. A method according to any of the preceding claims wherein a subject is considered to have an elevated probability of developing PE if the HbF-ratio exceeds 0.0020, such as e.g. 0.0025 such as e.g. 0.0060, such as e.g. 0.01.

By measuring and analyzing the HbF, HbA and the A1M level, the HbF the risk of developing PE may be assed by calculating the HbF-ratio and relating the HbF ratio to the A1M level. This may be done by e.g. logistic regression.

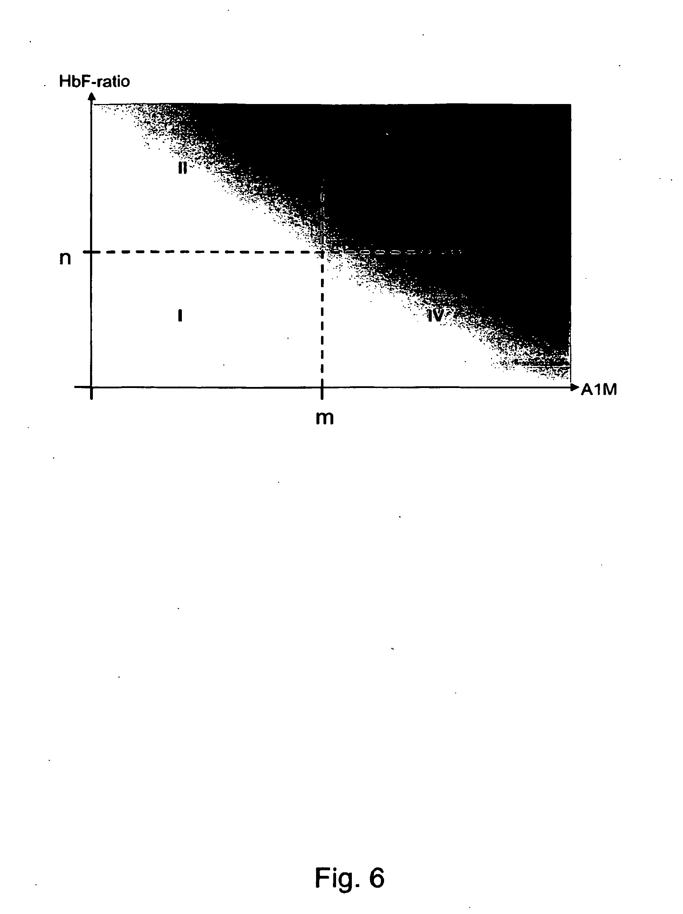

As outlined in figure 6, the HbF ratio may be used in combination with the A1 M-level to improve the prediction of developing of PE.

By combining the two parameters the precision of the prediction is greatly enhanced. As outlined in figure 6, specific reference values of the A1M level (value m, in figure 6) as well as the HbF ratio (value n, in figure 6) may be defined, by which subjects are considered as having elevated risk of developing PE is said reference values are exceeded.

In present invention, the reference value (m) is about 21µg/ml, such as e.g. 23µg/ml, such as e.g. 25µg/ml, such as e.g. 30µg/ml, such as e.g. 35µg/ml, such as e.g. 40µg/ml when a subject is considered having an elevated probability of developing preeclampsia. The reference value (n) is about 0.0020, such as e.g. 0.0025 such as e.g. 0.0060, such as e.g. 0.01. Thus, if one of the reference values is exceeded (represented as area II and IV), a subject is considered having an elevated risk of developing PE. If both values are exceeded (ie. area III, in figure 6) a subject is considered as having a highly elevated risk of developing PE.

Marker levels may be measured in samples isolated from a subject, most preferably maternal samples.

Samples may include blood, plasma, urine and serum/. Herein are described results derived from maternal venous serum/plasma. The levels may be measured by enzyme linked immunosorbent assay (ELISA), radioimmunoassay (RIA), Western blotting, spectrophotometry, *LC-MS, MALDI-TOF MS, chromatography* etc. Samples may also be from placenta or urine.

Samples may be isolated from a subject and analysed during the entire pregnancy period as example during gestational week 3-42, such as e.g. gestational week 7-24, such as e.g. gestational week 10-20 such as e.g. gestational week 11-16.

If possible, non invasive measurement on the subject may be perfomed during gestational week 3-42, such as e.g. gestational week 7-24, such as e.g. gestational week 10-20 such as e.g. gestational week 11-16.

The HbF level may be measured by any applicable method including chromatography, mass spectrometry and most preferably by enzyme linked immunosorbent arrays i.e. ELISA.

The A1M level may be measured by any applicable method including chromatography, mass spectrometry and preferably by enzyme linked immunosorbent arrays or radioimmunoassay (RIA).

The total Hb level as well as the HbA may be measured by any applicable method including chromatography, mass spectrometry and preferably by enzyme linked immunosorbent arrays or radioimmunoassay (RIA).

In a further embodiment, the invention relates to a kit for measuring and analyzing the level of A1 M and one more of the compounds HbA or HbF in a subject or in an isolated sample from a subject. Additionally to tools for measuring the A1M, HbA and HbF levels, the kit may comprise guidance on how to perform statistical analysis on the measured data, algorithms to perform the statistical analysis of the measured levels and reference values by which the measured and/or calculated values may be compared in order to assess the risk of developing PE. Further the kit may provide an automated device for measurement, calculation and PE risk assessment such e.g. a stationary or portable electronic device.

### DEFINITIONS

In this specification, unless otherwise specified, "a" or "an" means "one or more".

As used herein, PE is defined according to The International Society for the Study of Hypertension in Pregnancy. The definition for PE is hence: two readings of blood pressure greater than 140/90 mmHg at least four hours apart, and proteinurea, greater than or equal to 300 mg in 24 hours, or two readings of at least 2+ on dipstick analysis of midstream or catheter urine specimens, if no 24-hour urine collection was available. Alternatively, other definitions of the term "preeclampsia" may be applied as defined in accordance with e.g. the criteria established by the committee on Terminology of the American College of Obstetrics and Gynecology, that is, hypertension plus proteinuria, overt edema, or both. For example PE can be defined as blood pressure > 140/90 mm Hg and proteinuria > 0.3 g/L after 20 gestational weeks.

As defined herein, the term normal pregnancy was defined as delivery after 37 GW and normal blood pressure.

Hemoglobin A (HbA). There exist different forms of Hb. Adult Hb (HbA) consists of two alpha and two beta polypeptide chains (Hbα, Hbβ), each containing a non-peptide heme group that reversibly binds a single oxygen molecule. Hb A2, another adult Hb component is composed of two alpha chains and two delta chains (Hbα, Hbδ).

The term "free Hb", in this specification refers to free Hb generally and includes total free Hb, free Hb A, free Hb A2, free Hb F, any free Hb subunit (e.g. an Hbα, Hbβ, Hbδ or Hbγ chain), or any combination thereof. It further includes these Hb entities in either a polypeptide (protein) or nucleotide (RNA) form, except when applied as a target for treatment.

Fetal hemoglobin (HbF). The term "fetal Hb" refers to free Hb F or any subunit of Hb F and includes the Hb F entities in a polypeptide (protein) or nucleotide (RNA) form, except when applied as a target for treatment.

In this specification, the term "free" as used, inter alia, in the expressions "free Hb", "free fetal Hb" or "free Hb subunits (e.g. Hbα, Hbβ, Hbδ or Hbγ chains)" refer to Hb, fetal Hb or Hb subunits freely circulating in a biological fluid, as opposed to cellular Hb, which refers to the molecules residing inside cells. The term "free" in this sense is thus mainly used to distinguish free Hb from Hb, which is present in intact erythrocytes.

As used herein, the term referred to as HbF-ratio relates to HbF/total Hb and is calculated accordingly as the HbF level divided with the total Hb level.

The terms "marker" or "biomarker", in this specification, refer to a biomolecule, preferably, a polypeptide or protein, which is differentially present in a sample taken from a woman having PE or a woman at increased risk of developing PE as compared to a comparable sample taken from a woman, referred to as a "normal" woman/subject who does not have preeclampsia or is at increased risk of developing PE.

The term "biological sample from pregnant female mammal", the term "subject" or equivalents thereof are intended to denote a sample from the mammal itself; accordingly, the sample is not obtained from e.g. the fetus or the amniotic fluid.

As used herein fetal Hb abbreviated HbF refers to the type of Hb, which is the major component of Hb in the fetus. Fetal Hb has two alpha and two gamma polypeptide chains (Hbα, Hbγ).

As used herein α1-microglobulin (A1M), refers to the member of the lipocalin family named alpha-1-microglobulin. Alpha-1-microglobulin may be referred to in literature as A1M, EDC1, HCP, HI30, IATIL, ITI, ITIL, ITILC and UTI.

Throughout the specification, any and all references are specifically incorporated into this patent application by reference.

### BRIEF DESCRIPTION OF THE DRAWINGS

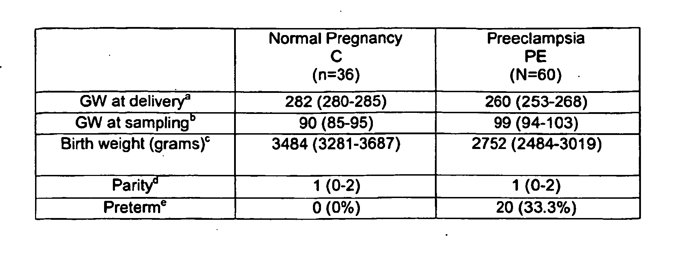

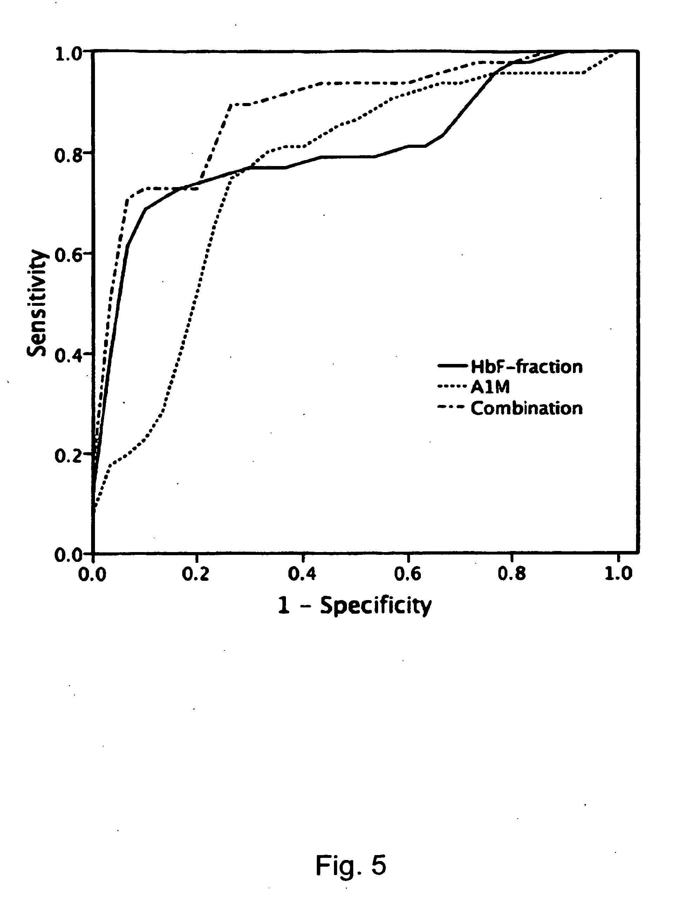

Figure 1. Demographics of cases and controls.
   Data for gestational week at delivery, at time of sampling, birth weight, parity and preterm delivery. Values are shown median (±2 standard errors) or number (%). Mann-Whitney U test was used except for ^{e}.
   ^{a}: p=0.000¹
   ^{b}: p=0.03
   ^{c}: p=0.001 after normalizing for gestational age.
   ^{d}: 27 primigravida amongst PE and 18 amongst normal pregnancies.
   ^{e}: Preterm defined as delivered before GW 37+0. 7 patients with PE delivered before GW 34+0.
Figure 2 shows the mean concentration of HbF, A1 M and total Hb in the preeclamptic cases and the controls. Binary logistic regression was used to determine the significance.
   ^{a}: Not calculated since the difference between the groups is not significant.
   ^{b}: HbF-ratio is defined as HbF-concentration/total Hb-concentration
   ^{c}: Odds ratio calculations based on HbF-ratio x 100. This is due to the very low values of HbF-fraction.
Figure 3. Sensitivity and specificity values for the HbF-ratio, A1M levels and the combination of the two parameters.
   ^{a}: HbF-ratio = HbF concentration/ total Hb concentration
   ^{b}: Based on logistic regression including both parameters.

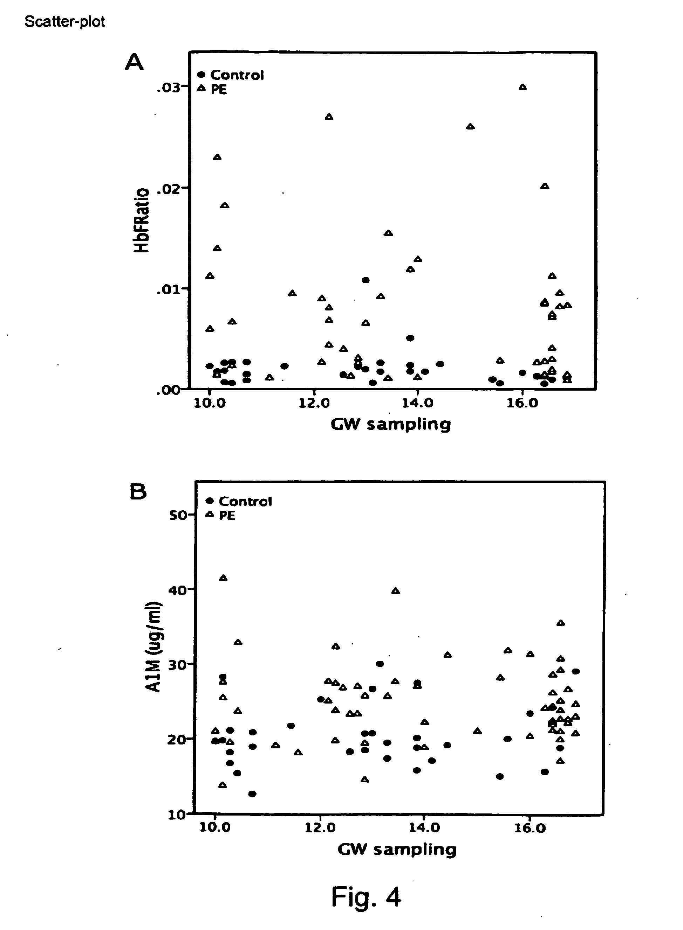

Figure 4. Scatter plot
   A: The scatter plot provides a graphical representation of the distribution of control samples and PE relative to the HbF-ration and the time of sampling.
   HbF-ratio= HbF/total Hb
   GW-sampling: the week of sampling
   B: The scatter plot provides a graphical representation of the distribution of control samples and PE relative to the A1M-level and the time of sampling.
   A1M: The A1M level measured in µg/ml
   GW-sampling: the week of sampling
Figure 5 Receiver Operating Characteristic (ROC) -curves.
   The ROC curves provide graphical representation of the Specificity (X-Axis) and Sensitivity (Y-axis) of the HbF-ratio, A1M-level and their combination.
Figure 6 Graphical representation of risk profile.
   m: reference value, A1M
   n: reference value, HbF-ratio
   Grey-scaled areas
   I: Low risk
   II and IV: Elevated risk
   III: Highly elevated risk.
   Color gradient, darkness represent risk.

### EXAMPLES

### Example 1

### Patients and demographics

A total of 96 women were included. Patients with other diseases such as diabetes and essential hypertension were excluded. Sixty women who subsequently developed PE (cases) and 36 with normal uncomplicated pregnancies (controls) were included. All controls delivered after 37+0 GW.

Characteristics are shown in Table 1. Of the 60 cases, 20 cases delivered before 37+0 gestational weeks (GW), of which seven delivered before 34+0 GW. All pregnancy outcomes were obtained from the main delivery suite database and checked for each individual patient. The International Society for the Study of Hypertension in Pregnancy definition for PE was used: two readings of blood pressure greater than 140/90 mmHg at least four hours apart, and proteinurea, greater than or equal to 300 mg in 24 hours, or two readings of at least 2+ on dipstick analysis of midstream or catheter urine specimens, if no 24-hour urine collection was available. Normal pregnancy was defined as delivery after 37 GW and normal blood pressure. The control samples were randomly selected from women who participated in the study during the same time period.

The patients were recruited with ethical permission, as part of an on-going prospective study of first trimester ultrasound and serum markers for PE in women attending a routine antenatal care visit at St Georges Hospital obstetric unit, London. Gestational age was calculated from the last menstrual period and confirmed by ultra sound crown-rump-length measurement.

### Example 2

### Sampling

A maternal venous serum sample was collected at 11 to 16 GW for analysis of HbF, total Hb and A1M. The venous blood was collected into a 5 ml vacutainer tube (Beckman Dickson) without additives to allow clotting, and centrifugated at 2000 g at room temperature for 10 min.

The serum was stored at -80 °C until further analysis.

### Example 3

### Measurement of total Hb, HbF and A1M

HbF and A1 M concentrations were determined by a sandwich ELISA and radioimmunoassay (RIA), respectively, as previously described. The total Hb concentration in serum was measured by a competitive ELISA, using antibodies against adult Hb (HbA) as previously described. The ratio HbF/total Hb was calculated and refered to as HbF-ratio.

### Example 4

### Statistical analysis

Statistical computer software (SPSS) was used to analyze the data. The probability of developing PE was analyzed in relation to the HbF-ratio and/or the HbF, HbA or A1M-levels, using a binary logistic regression analysis with a likelihood ratio (LR) test. Odds ratio (OR) was calculated for the biomarkers. Since the HbF-ratio showed low values it was multiplied by 100 before the OR was calculated. A significance level of 0.05 was used in all tests.

ROC curves for HbF, A1M, HbF-ratio and the combination of HbF-ratio and A1M were done based on the logistic regression results. The area under curve (AUC) was calculated from the ROC curves. The logistic regression analysis allowed us to test sensitivity at different screen positive rates. The optimal sensitivity level was evaluated. The linear correlations between the three biomarkers were evaluated by bivariate correlation analysis calculating the Pearson's correlation coefficients.

### Example 5

### The study groups

The demographic data of the included cases and the controls are shown in Figure 1 and Example 1. There was an expected, significant difference in gestational length at the time of delivery. There was a significant difference in birth weight after correlation to gestational age (p=0.001). The PE blood samples were on average collected 8 days later than the control samples (p=0.018).

### Example 6

### HbF-ratio and A1M plasma levels

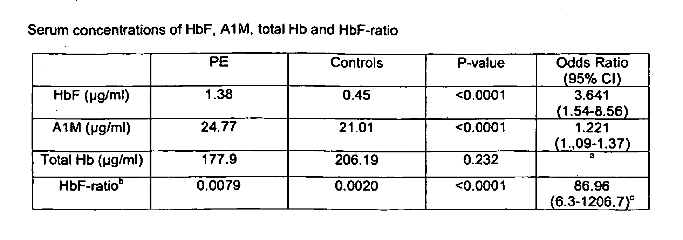

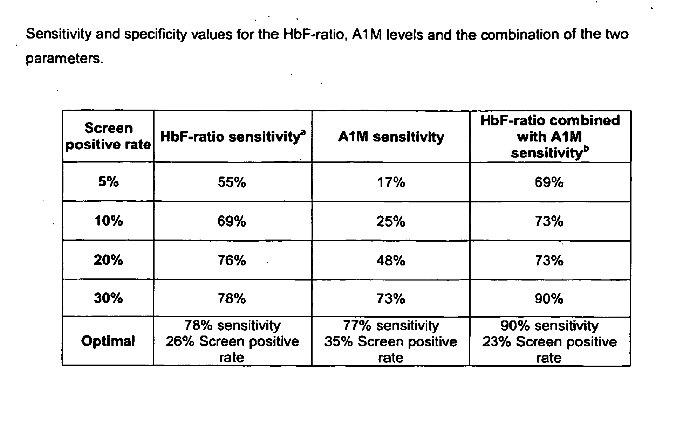

A high concentration of total Hb was seen in all samples, suggesting a certain degree of hemolysis. The high levels were verified by spectrophotometry (data not shown). However, there was no significant difference in total Hb concentration between the examined groups, 178µg/ml in PE and 206µg/ml in the controls (p=0.232) (Figure 2). The mean concentration of HbF was 1.38µg/ml in PE and 0.45µg/ml in the controls. Since background hemolysis may contribute to the HbF-values, and since HbF levels significantly correlated to Hb total levels, the HbF-ratio (HbF/total Hb) was calculated. The mean HbF-ratio was significantly elevated in PE compared to controls (p<0.0001)(Figure 2). The HbF-ratio related to the gestational age is shown in a scatter plot (Figure 3A). The mean A1M level was significantly elevated in PE (24µg/ml) compared to controls (21µg/ml, p=0.0001). The A1M levels are related to gestational age and shown in a scatter plot (Figure 3B).

### Example 7

### Correlation analysis

Neither the HbF levels nor the HbF-ratio levels were not significantly correlated to the A1M levels (p=0.17, r=0.16). However HbF levels significantly correlated to total Hb levels (p=0.001, r=0.35), a correlation that was even stronger in the PE cases (p=0.005, r=0.39). The levels of total Hb and A1M were not significantly correlated (p=0.61, r=-0.055).

### Correlation of the HbF-ratio to gestational age

The HbF-ratio and A1M levels were tested for correlation to gestational age at sampling (graphically presented in Figure 1). No correlation to gestational age was observed between 10-16 GW for neither HbF-ratio nor A1M.

### Logistic regression and receiver operating characteristic (ROC) curves

Logistic regression was used for calculating significance and odds ratios (Figure 2). ROC curves were drawn (Figure 5) and the sensitivity and specificity for the HbF-ratio, A1 M and their combination were obtained. The results were analyzed at four different cut-off values as presented in Figure 3. The area under the curve (AUC) was 0.82 for the HbF-ratio, 0.75 for A1M and 0.89 for the combination of the HbF-ratio and A1 M. The combination of the two markers showed the highest values of prediction, with an optimal sensitivity of 90% at 23% screen positive rate (Figure 3).

### Example 8

### Diagnosis by combined measurement of the HbF-ratio and the A1M level

HbF and A1 M concentrations were determined by a sandwich ELISA and radioimmunoassay (RIA), respectively, as previously described. The total Hb concentration in serum was measured by a competitive ELISA, using antibodies against adult Hb (HbA) as previously described. The ratio HbF/total Hb was calculated and refered to as HbF-ratio.

As outlined in figure 6, the HbF ratio may be used in combination with the A1M-level to improve the prediction of developing of PE.

By combining the two parameters the precision of the prediction is greatly enhanced. As outlined in figure 6, specific reference values of the A1 M level (value m, in figure 6) as well as the HbF ratio (value n, in figure 6) may be defined, by which subjects are considered as having elevated risk of developing PE if said reference values are exceeded.

In present invention, the reference value (m) is about 21µg/ml, such as e.g. 23µg/ml, such as e.g. 25µg/ml, such as e.g. 30µg/ml, such as e.g. 35µg/ml, such as e.g. 40µg/ml a subject is considered having an elevated probability of developing preeclampsia. The reference value (n) is about 0.0020, such as e.g. 0.0025 such as e.g. 0.0060, such as e.g. 0.01. Thus if one of the reference values is exceeded (represented as area II and IV), a subject is considered having an elevated risk of developing PE. If both values are exceeded (ie. area III, in figure 6) a subject is considered as having a highly elevated risk of developing PE.

## Claims

1. A method for predicting the risk of developing preeclampsia, the method being **characterized by** comprising the steps
a) During gestational week 10-20 measuring the levels of A1M, HbA and HbF in an isolated serum sample from a subject,
b) Calculating the ratio HbF/total Hb (HbF ratio),
wherein the subject is considered to have an elevated probability of developing preeclampsia if the measured HbF-ratio exceeds 0.0020 and the measured A1 M level exceeds 21 µg/ml.

2. A method according to claim 1, wherein the sample is a maternal sample.

3. A method according to claim 1 or 2, wherein the sample is isolated from a subject gestational week 11-16.

4. A method according to any of the preceding claims wherein the measurement is perfomed during gestational week 11-16.

5. A method according to any of the preceding claims in which HbF level is determined by ELISA

6. A method according to any of the preceding claims in which the A1 M level is determined by radioimmunoassay (RIA) or ELISA.

7. A method according to any of the preceding claims in which the total Hb concentration is measured by ELISA or competitive ELISA.

8. A method according to any of the preceding claims wherein the probability of developing preeclampsia is analyzed using a binary logistic regression analysis with a likelihood ratio (LR) test.

9. A method according to any of the preceding claims wherein a subject is considered to have an elevated probability of developing preeclampsia if the measured HbF level exceeds 0.45 µg/ml, 0.75 µg/ml, 1.0 µg/ml, 1.5 µg/ml or 2.5 µg/ml.

10. A method according to any of the preceding claims wherein a subject is considered to have an elevated probability of developing preeclampsia if the measured A1M level exceeds 23µg/ml, 25µg/ml, 30µg/ml, 35µg/ml, or 40µg/ml.

11. A method according to any of the preceding claims wherein a subject is considered to have an elevated probability of developing preeclampsia if the HbF-ratio exceeds 0.0025 0.0060, or 0.01.

12. A method according to any of the preceding claims wherein a subject is considered to have a highly elevated probability of developing preeclampsia if the HbF-ratio exceeds 0.0020, 0.0025 0.0060, or 0.01 and the A1M level exceeds 21µg/ml, 23µg/ml, 25µg/ml, 30µg/ml, 35µg/ml, or 40µg/ml.

13. Use of A1M, HbF and total Hb as early stage markers during gestational week 10-20 for predicting the possibility of a subject to develop preeclampsia, wherein the subject is considered to have an elevated probability of developing preeclampsia if the ratio HbF/total HB (HbF-ratio) exceeds 0.0020 and the measured A1M level exceeds 21 µg/ml.

14. Use according to claim 13, wherein the A1M, HbA and HbF levels are measured in an isolated serum sample.

15. Use according to any of claims 13-14, wherein the sample is isolated from a subject during gestational week 11-16.

16. Use according to any of claim 13-15, wherein a subject is considered to have an highly elevated probability of developing preeclampsia if the HbF-ratio exceeds 0.0020, 0.0025, 0.0060, or 0.01 and the A1M level exceeds 21µg/ml, 23µg/ml, 25µg/ml, 30µg/ml, 35µg/ml, or 40µg/ml.

## Patentansprüche

1. Ein Verfahren zum Prognostizieren des Risikos eine Präeklampsie zu entwickeln, das Verfahren **gekennzeichnet durch** die Schritte umfassend:
a) Während der 10. - 20. Schwangerschaftswoche Messung der A1M-, HbA- und HbF-Spiegel in einer isolierten Serumprobe von einem Subjekt,
b) Berechnung des Verhältnisses HbF/gesamt Hb (HbF-Verhältnis),
wobei das Subjekt befunden wird eine erhöhte Wahrscheinlichkeit zu haben Präehlampsie zu entwickeln, wenn das gemessene HbF-Verhältnis 0,0020 übersteigt und der gemessene A1M-Spiegel 21 µg/ml übersteigt.

2. Ein Verfahren nach Anspruch 1, wobei die Probe eine mütterliche Probe ist.

3. Ein Verfahren nach Anspruch 1 oder 2, wobei die Probe aus einem Subjekt in der 11. - 16. Schwangerschaftswoche isoliert wird.

4. Ein Verfahren nach einem der vorhergehenden Ansprüche, wobei die Messung während der 11. - 16. Schwangerschaftswoche durchgeführt wird.

5. Ein Verfahren nach einem der vorhergehenden Ansprüche, in dem HbF-Spiegel durch ELISA bestimmt wird.

6. Ein Verfahren nach einem der vorhergehenden Ansprüche, in dem der A1M-Spiegel durch Radioimmun-Assay (RIA) oder ELISA bestimmt wird.

7. Ein Verfahren nach einem der vorhergehenden Ansprüche, in dem die gesamte Hb-Konzentration durch ELISA oder kompetitiven ELISA gemessen wird.

8. Ein Verfahren nach einem der vorhergehenden Ansprüche, wobei die Wahrscheinlichkeit eine Präehlampsie zu entwickeln unter Verwendung einer binären logistischen Regressions-Analyse mit einem Wahrscheinlichkeitsverhältnis (LR)-Test analysiert wird.

9. Ein Verfahren nach einem der vorhergehenden Ansprüche, wobei ein Subjekt befunden wird eine erhöhte Wahrscheinlichkeit zu haben Präeklampsie zu entwickeln, wenn der gemessene HbF-Spiegel 0,45 µg/ml, 0,75 µg/ml, 1,0 µg/ml, 1,5 µg/ml oder 2,5 µg/ml übersteigt.

10. Ein Verfahren nach einem der vorhergehenden Ansprüche, wobei ein Subjekt befunden wird eine erhöhte Wahrscheinlichkeit zu haben Präeklampsie zu entwickeln, wenn der gemessene A1M-Spiegel 23 µg/ml, 25 µg/ml, 30 µg/ml, 35 µg/ml oder 40 µg/ml übersteigt.

11. Ein Verfahren nach einem der vorhergehenden Ansprüche, wobei ein Subjekt befunden wird eine erhöhte Wahrscheinlichkeit zu haben Präeklampsie zu entwickeln, wenn das HbF-Verhältnis 0,0025 0,0060 oder 0,01 übersteigt.

12. Ein Verfahren nach einem der vorhergehenden Ansprüche, wobei ein Subjekt befunden wird eine stark erhöhte Wahrscheinlichkeit zu haben Präehlampsie zu entwickeln, wenn das HbF-Verhältnis 0,0020, 0,0025 0,0060 oder 0,01 übersteigt und der A1M-Spiegel 21 µg/ml, 23 µg/ml, 25 µg/ml, 30 µg/ml, 35 µg/ml oder 40 µg/ml übersteigt.

13. Verwendung von A1M, HbF und gesamtem Hb als Frühstadiums-Marker während der 10. - 20. Schwangerschaftswoche zur Prognostizierung der Wahrscheinlichkeit eines Subjekts Präeklampsie zu entwickeln, wobei das Subjekt befunden wird eine erhöhte Wahrscheinlichkeit zu haben Präeklampsie zu entwickeln, wenn das Verhältnis HbF/gesamt HB (HbF-Verhältnis) 0,0020 übersteigt und der gemessene A1M-Spiegel 21 µg/ml übersteigt.

14. Verwendung nach Anspruch 13, wobei die A1M-, HbA- und HbF-Spiegel in einer isolierten Serum-Probe gemessen werden.

15. Verwendung nach einem der Ansprüche 13 - 14, wobei die Probe aus einem Subjekt in der 11. - 16. Schwangerschaftswoche isoliert wird.

16. Verwendung nach einem der Ansprüche 13-15, wobei ein Subjekt befunden wird eine stark erhöhte Wahrscheinlichkeit zu haben Präeklampsie zu entwickeln, wenn das HbF-Verhältnis 0,0020, 0,0025, 0,0060 oder 0,01 übersteigt und der A1M-Spiegel 21 µg/ml, 23 µg/ml, 25 µg/ml, 30 µg/ml, 35 µg/ml oder 40 µg/ml übersteigt.

## Revendications

1. Procédé destiné à prévoir le risque de développer une pré-éclampsie, le procédé étant **caractérisé par** les étapes consistant à :
a) au cours d'une semaine de gestation comprise entre la 10^{ème} et la 20^{ème} semaine, mesurer les niveaux d'A1M, d'HbA et d'HbF dans un échantillon de sérum isolé en provenance d'une patiente ;
b) calculer le rapport HbF/Hb total (rapport HbF) ;
dans lequel la patiente est considérée comme présentant une probabilité élevée de développer une pré-éclampsie si le rapport HbF mesuré dépasse 0,0020 et si le niveau d'A1M mesuré dépasse 21 µg/ml.

2. Procédé selon la revendication 1, dans lequel l'échantillon est un échantillon maternel.

3. Procédé selon la revendication 1 ou la revendication 2, dans lequel l'échantillon est isolé au cours d'une semaine de gestation d'une patiente comprise entre la 11^{ème} et la 16^{ème} semaine.

4. Procédé selon l'une quelconque des revendications précédentes, dans lequel la mesure est exécutée au cours d'une semaine de gestation comprise entre la 11^{ème} et la 16^{ème} semaine.

5. Procédé selon l'une quelconque des revendications précédentes, dans lequel le niveau d'HbF est déterminé selon une méthode ELISA.

6. Procédé selon l'une quelconque des revendications précédentes, dans lequel le niveau d'A1M est déterminé selon une méthode de radio-immuno-essai (RIA) ou selon une méthode ELISA.

7. Procédé selon l'une quelconque des revendications précédentes, dans lequel la concentration totale d'Hb est mesurée selon une méthode ELISA ou selon une méthode ELISA concurrentielle.

8. Procédé selon l'une quelconque des revendications précédentes, dans lequel la probabilité de développer une pré-éclampsie est analysée en utilisant une analyse de régression logistique binaire avec un test de rapport de vraisemblance.

9. Procédé selon l'une quelconque des revendications précédentes, dans lequel une patiente est considérée comme présentant une probabilité élevée de développer une pré-éclampsie si le niveau d'HbF mesuré dépasse 0,45 µg/ml, 0,75 µg/ml, 1,0 µg/ml, 1,5 µg/ml ou 2,5 µg/ml.

10. Procédé selon l'une quelconque des revendications précédentes, dans lequel une patiente est considérée comme présentant une probabilité élevée de développer une pré-éclampsie si le niveau d'A1M mesuré dépasse 23 µg/ml, 25 µg/ml, 30 µg/ml, 35 µg/ml ou 40 µg/ml.

11. Procédé selon l'une quelconque des revendications précédentes, dans lequel une patiente est considérée comme présentant une probabilité élevée de développer une pré-éclampsie si le rapport HbF mesuré dépasse 0,0025, 0,0060, ou 0,01.

12. Procédé selon l'une quelconque des revendications précédentes, dans lequel une patiente est considérée comme présentant une probabilité extrêmement élevée de développer une pré-éclampsie si le rapport HbF mesuré dépasse 0,0020, 0,0025, 0,0060 ou 0,01 et si le niveau d'A1M dépasse 21µg/ml, 23 µg/ml, 25 µg/ml, 30 µg/ml, 35 µg/ml ou 40 µg/ml.

13. Utilisation d'A1M, d'HbF et d'Hb total en tant que marqueurs de stade précoce au cours d'une semaine de gestation comprise entre la 10^{ème} et la 20^{ème} semaine afin de prévoir la possibilité que présente une patiente de développer une pré-éclampsie, dans laquelle la patiente est considérée comme présentant une probabilité élevée de développer une pré-éclampsie si le rapport HbF/Hb total (rapport HbF) dépasse 0,0020 et le niveau d'A1M mesuré dépasse 21 µg/ml.

14. Utilisation selon la revendication 13, dans laquelle les niveaux d'A1M, d'HbA et d'HbF sont mesurés dans un échantillon de sérum isolé.

15. Utilisation selon la revendication 13 ou la revendication 14, dans lequel l'échantillon est isolé au cours d'une semaine de gestation d'une patiente comprise entre la 11^{ème} et la 16^{ème} semaine.

16. Utilisation selon l'une quelconque des revendications 13 à 15, dans lequel une patiente est considérée comme présentant une probabilité extrêmement élevée de développer une pré-éclampsie si le rapport HbF mesuré dépasse 0,0020, 0,0025, 0,0060 ou 0,01 et si le niveau d'A1M dépasse 21 µg/ml, 23 µg/ml, 25 µg/ml, 30 µg/ml, 35 µg/ml ou 40 µg/ml.
